**Europäisches Patentamt**

**European Patent Office**

**Office européen des brevets**

(19)

(11) Veröffentlichungsnummer: **0 177 755**
**B1**

(12) # EUROPÄISCHE PATENTSCHRIFT

(45) Veröffentlichungstag der Patentschrift:
02.11.88

(51) Int. Cl.⁴: **A 61 F 2/38**

(21) Anmeldenummer: **85111162.5**

(22) Anmeldetag: **04.09.85**

(54) Tibiaprothesenteil einer Kniegelenk-Endoprothese.

(30) Priorität: 07.09.84 DE 3432929

(43) Veröffentlichungstag der Anmeldung:
16.04.86 Patentblatt 86/16

(45) Bekanntmachung des Hinweises auf die Patenterteilung:
02.11.88 Patentblatt 88/44

(84) Benannte Vertragsstaaten:
AT BE CH FR GB IT LI LU NL SE

(56) Entgegenhaltungen:
EP-A-0 174 531
CH-A-613 111
DE-A-3 013 155
GB-A-1 509 366
US-A-4 257 129

(73) Patentinhaber: S + G IMPLANTS GMBH,
Grapengiesserstrasse 21, D-2400 Lübeck (DE)

(72) Erfinder: Grundei, Hans, Gärtnergasse, D-2400
Lübeck (DE)

(74) Vertreter: Wilcken, Thomas, Dipl.- Ing.,
Musterbahn 1, D-2400 Lübeck (DE)

EP 0 177 755 B1

**Beschreibung**

Die Erfindung bezieht sich auf eine Kniegelenkendoprothese nach dem Oberbegriff des Anspruches 1.

Bei Tibiaprothesenteilen mit Gleitflächen für die Kufen des Femur-Prothesenteils, z. B. nach der DE-PS-2 549 819, tritt mit der Zeit ein Verschleiß der Gleitflächen auf, so daß es erforderlich wird, den Tibiateil mit diesen Gleitflächen zu ersetzen. Hierzu war es erforderlich, den gesamten Prothesenteil mit Stiel- und Tibiateil operativ vom Schienbein zu lösen und dann eine Prothese mit neuen Tibiagleitflächen wieder zu implantieren.

Unter Verwendung von Stielen, die eine durchgehende oder in der Außenschicht offenzellige, metallische Struktur aufweisen, wie sie z. B. nach der DB-PS-3 106 917 herstellbar sind, besteht die Aufgabe der Erfindung darin, die Tibiagleitflächen nach Verschleiß bei Prothesen entsprechend der DE-PS-2 549 819 schnell und einfach ohne operativen Eingriff lösen und ersetzen zu können.

Diese Aufgabe wird nach der Erfindung dadurch gelöst, daß der metallische Stiel des Tibiateiles mit einer bekannten Außenschicht aus einer offenzelligen metallischen, die Bildung von Knochen in den Zellen zulassenden Struktur und mit einer oberen ebenen, einen mittigen bis zu einem dorsalen Anschlag verlaufen den Ausschnitt aufweisenden Stützfläche versehen ist, unterhalb welcher beidseitig des Ausschnittes nutförmige Führungen für den Eingriff unterer Flansche des Mittelsteges vorgesehen sind und daß der Mittelsteg an seinen lateralen Seiten von hochgezogenen Wänden und an seiner ventralen Seite von einem Verbindungsteil eines aus Kunststoff bestehenden Tibiaprothesenteiles eingefaßt ist, welches Tibiaprothesenteil weiterhin auf der Stützfläche aufruhende, ausgekehlte Gleitflächen für die beiden Kufen des Femurteiles aufweist, wobei die hochgezogenen Wände des Tibiaprothesenteiles von dem Querzapfen durchgriffen werden und wobei der Mittelsteg durch einen lösbaren Stift gegenüber der Stützfläche festgelegt ist.

Damit ist es möglich, die Gleitflächen des Tibiateils bzw. den gesamten oberen Tibiateil ohne Ausbau des im Schienbein verankerten Stieles auszutauschen wie noch im einzelnen erläutert wird.

Durch die Lösung nach der Erfindung bleibt der Stiel der Tibiaprothese ständig im Schienbein. Er ist, wie sich in der Praxis gezeigt hat, durch das Einwachsen von Knochengewebe mit anschließender Knochenbildung in die Poren oder Zellen des Belages eine solch innige Verbindung mit dem Scheinbein eingegangen, daß ein Herauslösen des Stieles nur durch Beschädigungen des Schienbeines möglich wäre. Es ist damit die lösbare Verbindung des Tibiateiles vom Femurteil entscheidend, um die Gleitflächen des Tibiateiles austauschen zu können.

Die Erfindung wird nachstehend anhand der Zeichnung erläutert.Es zeigen:

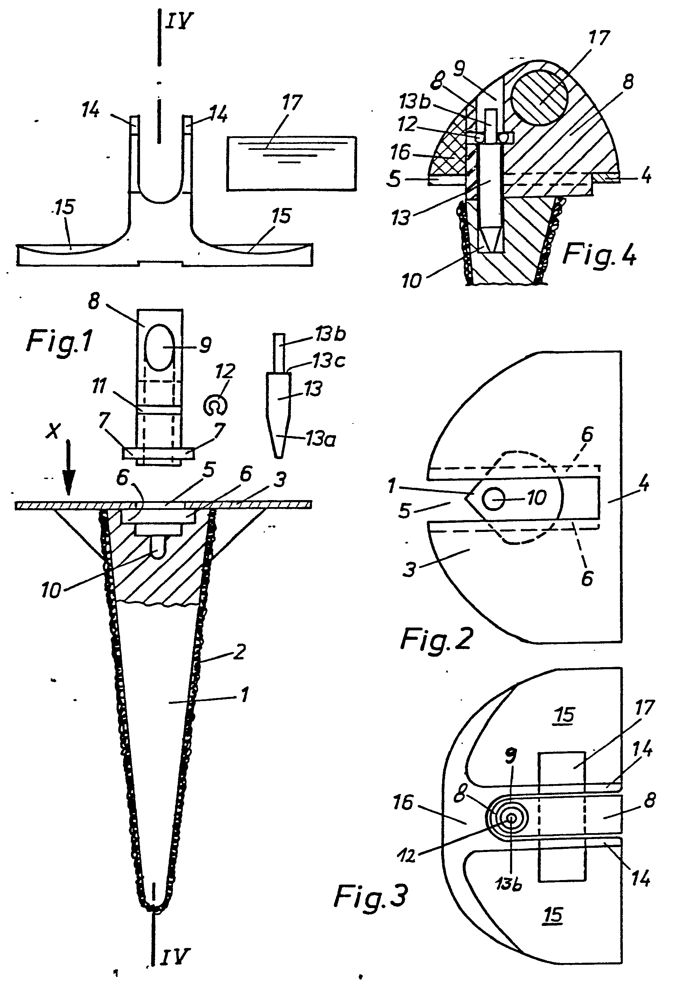

Figur 1    die auseinander gezogen dargestellten Teile einer Tibiaendoprothese nach der Erfindung in Stirnansicht und teilweisem Schnitt durch das oberteil des Stieles,

Figur 2    eine Aufsicht auf die obere ebene Stützfläche des Stieles,

Figur 3    eine Aufsicht auf den Tibiaprothesenteil mit den oberen Gleitflächen,

Figur 4    einen senkrechten Längsschnitt durch die Tibiaendoprothese.

Die Tibiaendoprothese besteht aus einem metallischen Stiel und dem mit ihm verbindbaren Tibiateil. Der Stiel 1 besitzt eine Außenschicht 2 offenzelliger, metallischer Struktur und ist oben mit einer ebenen Stützfläche 3 versehen. Der Stiel 1 kann aber auch durchgängig offenzellig ausgebildet sein. Die Stützfläche 3 ist mit einem von vorn nach hinten bis zu einem Anschlag 4 verlaufenden Ausschnitt 5 versehen, der beidseitig nutförmige Führungen 6 mit der Stieloberseite bildet. In diese Führungen 6 greifen die unteren Seitenflansche 7 eines metallischen Mittelsteges 8, der von vorn in den Ausschnitt 5 eingeführt wird. Der Mittelsteg 8 ist etwa in Richtung der Stielachse mit einer aufrechten Durchbohrung 9 versehen, unter der der Stiel 1 eine Einbohrung 10 besitzt. Im Bereich der Durchbohrung 9 ist der Mittelsteg 8 vorn mit einem waagerechten Einschnitt 11 versehen, in den ein gespreizter Federring 12 einschiebbar ist, bis seine Mitte in der Achse der Durchbohrung 9 liegt. Es wird nun ein Fixierstift 13 in die Durchbohrung 9 eingesetzt, der durch Druck von oben mit seinem unteren Konus 13a den Federring 12 spreizt, bis er teilweise gegen eine obere, das Oberende 13b umgebende Schulter 13c liegt. Dabei greift der Konus 13a in die Bohrung 10 des Stieles 1 ein. Damit ist der Mittelsteg 8 auf der oberseite des Stieles 1 fixiert.

Der Mittelsteg 8 wird von zwei nach oben gezogenen Wänden 14 der beiden aus Kunststoff bestehenden, ausgekehlten, auf der Stützfläche 3 aufruhenden Gleitflächen 15 seitlich und durch einen vorderen Verbindungsteil 16 der Gleitflächen eingefaßt. Die Wände 14 und der Mittelsteg 8 besitzen Querdurchbohrungen zum Durchführen eines Lagerzapfens 17, durch den die Tibiateile 14, 15 mit dem auf der Stützfläche 3 fixierten Mittelsteg lösbar verbunden werden. Um die freien Enden des Lagerzapfens 17 sind die Kufen des Femurteils einer Kniegelenkendoprothese schwenkbar, wie in der DE-PS-2 549 819 beschrieben und dargestellt ist.

Nach einem etwaigen Verschleiß der Gleitflächen 15 wird die vordere Gleitflächenverbindung 16 in Längsrichtung bis zum Mittelsteg 8 durchtrennt, so daß die beiden Gleitflächen 15 nach Trennung des Tibiateiles vom Femurteil der Kniegelenkendoprothese von den Enden des Lagerzapfens 17 herabgezogen und ausgetauscht werden können.

Nach Entnahme eines zu ersetzenden Tibiateils 14, 15, 16 kann auch der Mittelsteg 8 vom Stiel 1 gelöst werden, und zwar dadurch, daß auch der geschlitzte Federring 12 freigeworden ist, der nun nach vorn herausgleiten kann, so daß nunmehr auch der Fixierzapfen 13 nach oben herausziehbar ist. Damit kann der Mittelsteg 8 nach vorn geschoben und gelöst werden.

Die Breite des Federringschlitzes ist im übrigen größer gewählt als der Durchmesser des Endes 13b des Fixierzapfens 13, so daß der Federring 12 nach Entfernen der Tibiagleitflächen aus dem Schlitz 11 leicht heraus fallen kann.

## Patentansprüche

1. Kniegelenk-Endoprothese umfassend einen durch einen metallischen Stiel (1) im Schienbein zu verankernden Tibiateil mit einem metallischen Mittelsteg (8) und einen den Mittelsteg (8) übergreifenden, bekannten Femurteil, der miteinander zugekehrten Führungsnuten die beiden Enden eines im Mittelsteg gelagerten Querzapfens (17) übergreift, welcher exentrisch zum Stiel nach dorsal verlagert ist, dadurch gekennzeichnet, daß der metallische Stiel (1) des Tibiateils mit einer bekannten Außenschicht aus einer offenzelligen, metallischen, die Bildung von Knochen in den Zellen zulassenden Struktur und mit einer oberen, ebenen, einen mittigen bis zu einem dorsalen Anschlag (4) verlaufenden Ausschnitt (5) aufweisenden Stützfläche (3) versehen ist, unterhalb welcher beidseitig des Ausschnittes (5) nutförmige Führungen (6) für den Eingriff unterer Flansche (7) des Mittelsteges (8) vorgesehen sind, und daß der Mittelsteg (8) an seinen lateralen Seiten von hochgezogenen Wänden (14) und an seiner ventralen Seite von einem Verbindungsteil (16) eines aus Kunststoff bestehenden Tibiaprothesenteiles (14, 15, 16) eingefaßt ist, welches Tibiaprothesenteil weiterhin auf der Stützfläche (3) aufruhende, ausgekehlte Gleitflächen (15) für die beiden Kufen des Femurteiles aufweist, wobei die hochgezogenen Wände (14) des Tibiaprothesenteiles von dem Querzapfen (17) durchgriffen werden und wobei der Mittelsteg (8) durch einen lösbaren Stift (13) gegenüber der Stützfläche (3) festgelegt ist.

2. Kniegelenk-Endoprothese nach Anspruch 1, dadurch gekennzeichnet, daß der mit den Gleitflächen (15) durch den Querzapfen (17) verbundene Mittelsteg (8) eine aufrechte vordere Durchbohrung zur Aufnahme des lösbaren Stiftes (13) aufweist, dessen Unterende in eine obere Einbohrung (10) des Stieles (1) eindrückbar ist und in der Durchbohrung durch einen geschlitzten Federring (12) festgehalten ist, der von vorn durch eine waagerechte Nut (11) des Mittelsteges (8) einführbar ist und axial gegen eine Schulter (13c) des Zapfens (13) liegt.

3. Kniegelenk - Endoprothese nach Anspruch 2, dadurch gekennzeichnet, daß der Mittelsteg (8) eine aufrechte Durchbohrung (9) zur Aufnahme des Fixierzapfens (13) aufweist, dessen Unterende in eine obere Einbohrung (10) des Stieles (1) eindrückbar ist und in der Durchbohrung durch einen geschlitzten, gegen eine obere Schulter (13c) des Fixierzapfens zur Anlage kommenden Federring (12) festgelegt ist.

## Claims

1. Knee joint endoprosthesis comprising a tibia element which is to be anchored in the tibia by means of a metal shank (1), with a central bridge (8) and a known femur element engaging over the central bridge (8) which engages with mutually opposed guiding grooves over both ends of a transverse pin (17) housed in the central bridge, which is eccentrically offset in the dorsal direction with respect to said shank, characterised in that the metal shank (1) of the tibia element is provided with a known external layer of an open-celled metal structure allowing the formation of bone in the cells and with an upper plane support surface (3) having a central excision (5) extending up to a dorsal stop (4), below which and on both sides of the excision (5) there are provided groove-like guides (6) for the engagement of bottom flanges (7) of the central bridge (8), and that the central bridge (8) is enflanked on its lateral sides by raised walls (14) and on its ventral side by a coupling element (16) of a tibia prosthesis element (14, 15, 16) consisting of plastics material, which tibia prosthesis element moreover has concavely formed sliding surfaces (15) for the two rockers of the femur element resting on the supporting surface (3), wherein the raised walls (14) of the tibia prosthesis element are transversed by the transverse pin (17) and wherein the central bridge (8) is located with respect to the supporting surface (3) by means of a releasable peg (13).

2. Knee joint endoprosthesis according to claim 1, characterised in that the central bridge (8) joined to the sliding surfaces (15) by the transverse pin (17) has an upright front through-hole to receive the releasable peg (13) the lower end of which is insertible into an upper bore (10) of the shank (1) and is retained in the through-hole by means of a slotted annular spring (12) which is insertible from the front through a horizontal groove (11) of the central web (8) and bears axially against a shoulder (13c) of the peg (13).

3. Knee joint endoprosthesis according to claim 2, characterized in that the central bridge (8) comprises a vertical through-hole (9) for receiving the releasable peg (13) the lower end of which is insertible into an upper bore (10) of the shank (1) and is located in the through-hole (9) by means of a slotted annular ring (12) against which abuts an upper shoulder (13c) of said releasable peg.

## Revendications

1. Endoprothèse d'articulation du genou comprenant une partie tibiale à ancrer par une tige métallique (1) dans le tibia avec un pont métallique (8) et une partie fémorale connue s'engageant sur le pont (8), par des rainures de guidage adjacentes s'engageant sur les deux extrémités d'un tenon transversal (17) disposé dans le pont, et qui est disposée de manière excentrique par rapport à la tige dans la direction dorsale, caractérisée en ce que la tige métallique (1) de la partie tibiale est munie d'une couche extérieure connue faite d'une structure métallique à cellules ouvertes permettant la formation d'os dans les cellules, et d'une surface d'appui (3) supérieure plane présentant une encoche (5) s'étendant au milieu jusqu'à une butée dorsale (4), et sous laquelle des deux côtés de l'encoche (5) sont prévues des rainures de guidage (6) pour l'engagement de la collerette inférieure (7) du pont (8), et en ce que le pont (8) est entouré sur ses côtés latéraux par des parois dressées vers le haut (14) et sur son côté ventral par une pièce de liaison (16) d'une partie de prothèse tibiale faite de matière synthétique (14, 15, 16), laquelle partie de prothèse tibiale présente en outre des surfaces de glissement (15) cannelées reposant sur la surface d'appui (3) pour les deux patins de la partie fémorale, les parois dressées vers le haut (14) de la partie de prothèse tibiale (14) étant traversées par le tenon transversal (17) et le pont (8) étant fixé par une goupille amovible (13) contre la surface d'appui (3).

2. Endoprothèse d'articulation du genou selon la revendication 1, caractérisée en ce que le pont (8) relié aux surfaces de glissement (15) par le tenon transversal (17) présente un alésage avant droit pour l'engagement de la goupille amovible (13), dont l'extrémité inférieure peut être enfoncée dans un alésage supérieur de la tige (1) et est maintenue dans l'alésage par un anneau-ressort fendu (12) qui peut être introduit par l'avant par une rainure horizontale (11) du pont (8) et s'appuie axialement contre un épaulement (13c) de la goupille (13).

3. Endoprothèse d'articulation du genou selon la revendication 2, caractérisée en ce que le pont (8) présente un alésage droit (9) pour l'engagement de la goupille de fixation (13), dont l'extrémité inférieure peut être enfoncée dans un alésage supérieur (10) de la tige (1) et est maintenue dans l'alésage par un anneau-ressort (12) fendu, venant s'appliquer contre un épaulement supérieur (13c) de la goupille de fixation.

0 177 755

Fig.1

Fig.2

Fig.3

Fig.4

1